(19) **Europäisches Patentamt / European Patent Office / Office européen des brevets**

(11) **EP 2 047 805 A1**

(12) **EUROPEAN PATENT APPLICATION**
published in accordance with Art. 153(4) EPC

(43) Date of publication:
**15.04.2009 Bulletin 2009/16**

(51) Int Cl.:
*A61B 17/00* (2006.01)    *A61B 19/00* (2006.01)

(21) Application number: **07788684.4**

(22) Date of filing: **18.07.2007**

(86) International application number:
**PCT/ES2007/000442**

(87) International publication number:
**WO 2008/012386 (31.01.2008 Gazette 2008/05)**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **28.07.2006 ES 200602091**

(71) Applicant: **Universidad de Málaga**
**29071 Málaga (ES)**

(72) Inventors:
- **MUÑOZ MARTINEZ, Victor Fernando**
  **E-29071 Málaga (ES)**
- **GARCIA MORALES, Isabel**
  **E-29071 Málaga (ES)**
- **FERNANDEZ LOZANO, Jesus**
  **E-29071 Málaga (ES)**

- **GOMEZ DE GABRIEL, Jesus Manuel**
  **E-29071 Málaga (ES)**
- **GARCIA CEREZO, Alfonso**
  **E-29071 Málaga (ES)**
- **PEREZ DEL PULGAR, Carlos Jesus**
  **E-29071 Málaga (ES)**
- **SERON BARBA, Javier**
  **E-29071 Málaga (ES)**
- **DOMINGUEZ FERMANDEZ, Francisco**
  **E-29071 Málaga (ES)**
- **VARA THORBECK, Carlos**
  **E-29071 Málaga (ES)**
- **TOSCANO MENDEZ, Rafael**
  **E-29071 Málaga (ES)**

(74) Representative: **Carvajal y Urquijo, Isabel et al**
**Clarke, Modet & C.**
**C/ Goya, 11**
**E-28001 Madrid (ES)**

(54) **ROBOTIC SYSTEM FOR ASSISTING IN MINIMALLY-INVASIVE SURGERY, WHICH CAN POSITION A SURGICAL INSTRUMENT IN RESPONSE TO ORDERS FROM A SURGEON, IS NOT ATTACHED TO THE OPERATING TABLE AND DOES NOT REQUIRE PRE-CALIBRATION OF THE INSERTION POINT**

(57) Summary

Robotic system for assisting in minimally-invasive surgery, which can position a surgical instrument in response to orders from a surgeon, is not attached to the operating table and does not require pre-calibration of the insertion point. The system includes: a manipulator robot having three active degrees of freedom, which is provided with an end actuator having two passive degrees of freedom, said actuator being used to attach a surgical instrument; a robot controller built into the struc-
ture thereof, which can perform a method for calculating the movement to be imparted to the carried surgical instrument so that it reaches the desired location without requiring pre-calibration and without the assembly having to be attached to the operating table; and an interface system for ordering the system to perform the desired actions. The assembly comprising the robot, the controller and the interface system is battery operated.

EP 2 047 805 A1

**Description**

Field of the invention

The present invention relates to the field of surgery and robotics, specifically to the field of support systems for surgery, and more precisely, to manipulative robots specifically designed as surgical instruments.

Background of the invention

Surgeries using laparoscopic techniques consist of performing the operation through small incisions in the patient's abdomen. The required instruments for the particular operation are inserted through these incisions, as well as the optic of the camera which enables the surgeon to see the area where the operation is to be performed. This technique allows to drastically reduce the size of the incisions needed, which results in fewer risks for the patient, shorter postoperative periods and lower costs of the operation, as well as a smaller aesthetic impact.

Nowadays the normal procedure in these operations consists of holding the camera with the help of an assistant, while the surgeon uses the surgical set of instruments inserted in the patient's abdomen. This requires great coordination between the assistant and the surgeon, which, as successful as it may be, always has three fundamental problems:

1) The surgeon has to clearly transmit at every instant what it is that s/he wants the assistant to do. This is subject to all the problems of verbal communication, and the results expected by the surgeon are not always attained. As great as the understanding between the surgeon and his/her assistant may be, there are times when the surgeon does not clearly explain what s/he wants or the assistant misinterprets the surgeon's commands.

2) The image, as the camera is being held by a person, is not totally stable, being therefore affected by the pulse of the assistant handling it. This effect increases as the operation extends in time.

3) The assistant, being in an uncomfortable position, has difficulties to move the camera with precision, especially when s/he is affected by tiredness. This causes the camera optics to occasionally rub some viscera, which in turn makes it necessary to remove it and clean it. This problem extends the operation time and therefore, the time the patient is under anesthetic, which increases the risks inherent to it.

Besides, there are times when the surgeon needs to use more than two instruments at the same time, which requires a second surgeon to take part in the operation. This further aggravates the first of the aforementioned coordination problems.

Furthermore, the insertion points of the surgical instruments in the abdomen cannot be laterally modified, which limits the mobility of said instruments to two rotations around the insertion point, one around the axis of the tool and a movement along said axis. The nature of these movements poses a series of problems for the handling:

1) Inversion of movement. The insertion point acts as a fulcrum, making the tool to pivot around it (except in the penetration-extraction movement). Thus, a movement to the right of the surgeon's hand results in a movement to the left of the end of the instrument.

2) Scale. As if it were a lever, the tool amplifies or reduces the surgeon's movement according to the penetration. For a penetration over a particular value, there is amplification; below that value, there is reduction. It is worth pointing out that this effect does not limit itself to movements but also includes the forces exerted.

3) Loss of sense of touch. The textures of tissues and efforts, extremely helpful to surgeons in open surgery procedures, are transmitted in a very limited way in endoscopic techniques, as direct contact with the tissues is lost. Besides, the fulcrum and friction effect on the trocar distort and filter the little information of this kind to which the surgeon has access.

Like the visual problems (such as reduction of the field of vision, lost of image depth, lighting changes, etc.), the problems posed by handling in minimally-invasive surgery can be largely overcome through intense training. However, in the same way, this persuades certain surgeons of incorporating these techniques.

As at least a partial solution to these inconveniences, there have been proposed lately different robotized support systems for laparoscopic surgery (for example, in US5815640 or US6371952), to assist the surgeon in some of his/her tasks, such as handling the laparoscopic camera. These systems are designed according to one of the following strategies:

1) A mechanical structure of such a nature that its kinematics forces the surgical instrument held to move around a remote rotation center which is made to coincide with the insertion point of the instrument in the patient's abdomen. This is attained using a calibration procedure before the participation of the robotized system in the operation starts (for example, US6371952).

2) An end actuator based on passive joints, so that by positioning this end actuator and the support point implied by the insertion point of the instrument in the patient's abdomen, said instrument remains fixed and, ideally, positioned and oriented as desired. The existence of passive joints guarantees that regardless of the movement commanded to the instrument, no lateral forces are exerted to the insertion point even with inaccurate knowledge of the location of said point. However, since the orientation of the instrument is not directly controlled, but rather only the position adopted in space by the end actuator holding it, it is necessary to know the location of the insertion point of the tool in order to be able to accurately locate it as the surgeon desires. Said location is calculated using geometrical methods, physically fixing the robot to the operating table to limit estimate errors and avoid changes in the relative positions of the robot and the insertion point during the operation (for example, US5815640).

There is a certain number of precedents of robotic systems for surgery designed according to one of the preceding strategies with the purpose of improving the surgical practice. For example, ES2150880 presents a manipulating industrial robot modified to take part in laporoscopic surgery. The modifications of the robot consist in a mobile assembly with wheels which enables to conveniently place it in the operating room and an end actuator with two passive joints, together with a computer that coordinates and acts as a person-machine interface. Besides, it has a calculation procedure of the pivot point (the insertion point of the laparoscopic tool held) through an initial calibration. However, the volume and complexity of the assembly limit the application of this system.

EP0571827 presents a robotic surgical system based on a remote rotation center. As such, it has the inconvenience of requiring careful initial calibration to guarantee that the insertion point of the tool coincides with the remote rotation center of the robot mechanism. This inconvenience greatly hinders the use of robots in operations where the insertion point of the tool held needs to be changed, for example when the operation is for more than one illness (inguinal hernia and colecistectomy, to name a frequent case).

In ES2181526, the object of the patent is a surgical robot with a specific design, based on the transmission of movement using pulling ropes, and a total of seven degrees of freedom (with only three actuators). It also includes the possibility of receiving orders on the Internet, which entails a basic remote surgery system. Furthermore, it offers the possibility of registering the positions the robot adopts during the operation. In spite of all this, and besides the complexity of the system of movement transmission through cables, it still needs an initial calibration procedure of the pivot point just like the one shown in ES2150880.

W09403113 describes a robotic system for surgery based on passive joints, which has four active joints (and therefore four engines). It has two important limitations: the robot has to be anchored to the operating table and an initial calibration procedure has to be carried out before the operation to determine the location of the insertion point of the tool. The same limitations can be found in W09729690, where a remote surgery system is presented, which is based on the manipulative robot presented in W09403113.

US5784542 describes a bilateral remote surgery system where a direct correspondence is established between a tool attached to a robot and a joystick handled by a human operator. The system is used for microsurgery, where remote surgery is used as a means to increase the skill of the surgeon present on the operating room and not as a means to allow the intervention of a surgeon at a site remote from the patient. Its architecture of remote surgery does not contemplate this possibility and largely depends on real time communication between the different elements of the system, which would be unrealistic in the aforementioned case of a remote surgeon.

W09825666 describes a surgical system of telepresence, with a similar approach to US5784542, but where the surgeon receives more information of the surgical field, in the search of an *immersion* effect. It only partially contemplates the case in which the surgeon is at a remote site, insofar as the communication requirements of the system require the use of dedicated networks, and dismisses the use of general networks such as the Internet.

ES2203318 presents a robot remote surgery system where the robot component is a manipulative device of a specific design, with a different configuration from those of ES2150880 and ES2181526 (consisting in this case of three active degrees of freedom plus two passive degrees). This manipulative device also has wireless features (battery, microphones), and it is integrated in a remote surgery system, through modules with different functions and physical locations, and with graphic information exchange between the surgeon in the operating room and the surgeon at a remote site. A similar system, but based on a manipulative industrial robot and dedicated to the transurethral removal of the prostrate, is described in ES2200679.

To summarize, the state of the art presents several limitations:

1) Fixing the assistant robot to the operating table requires the modification of said table, which limits the impact of the robotized assistance systems and their spreading.

2) Fixing the assistant robot to the operating table hinders or even prevents its extraction in case of its malfunction or if its use is unnecessary (such as in the case of converting the surgical procedure from laparoscopy to laparotomy or conventional "open" surgery).

3) The requirement of a calibrating procedure before the operation for the system to know the location of the insertion point (in both strategies) includes an additional task in surgical operations, which can extend the operation time (during which the patient is under anesthetic) or reduce the advantage that can be drawn from using an assistant robot.

4) The need for previous calibration implies that if during the operation it is necessary to insert the instrument through an insertion point other than the initial one (which is usual when the patient undergoes more than one surgery during the same operation), it is necessary to repeat said calibration to find out the location of the new insertion point, which further extends the operation and limits the versatility of the robotic systems.

Description of the invention

The present document describes a robotic system which enables to securely handle a laparoscopic camera in minimally-invasive surgery without physically fixing the system to the operating table or previously calibrating the insertion point of the camera in the patient's abdomen, through the general configuration of the different degrees of freedom of the robot arm, and the general configuration of the overall robotized system itself, and an appropriate method to calculate the movement which has to be given to the camera for it to reach the desired location without any need to physically fix it to the operating table or a calibration procedure before the system starts to intervene in the surgery.

The system comprises a manipulative robot with three active degrees of freedom and an end actuator with two passive degrees of freedom specially designed to hold a camera for laparoscopic surgery, a controller for the integrated robot in its structure capable of implementing a method that calculates the movement to be given to the camera for it to reach the desired location without need for previous calibration or fixing the assembly to the operating table, and an interface system to order the desired actions to the system.

The end actuator is designed so that it adds two passive degrees of freedom to the robot. This makes the movement of the laparoscopic tool inside the patient's abdomen safer, since the use of the surgical instruments normally used in laparoscopy (when used directly by surgeons) is based on movements around a pivot point constituted by the place where the pivot point is inserted in the patient's skin. The use of these two passive joints enables the pivot point to be established naturally by the movement of the instrument, as it occurs when the tool is handled by a person, against what would happen if the instrument were directly fixed to the wrist of the robot, as establishing the pivot point would be determined by the calculations made to estimate it. In this case, any error would result in the robotic system tending to force the pivot point from its real location to the estimate one pushing the patient's skin. According to the magnitude of this error, a failure in calculating this pivot point could give rise to dangerous situations, such as tears in the patient's skin. By contrast, in the case of a system including passive joints, the error when calculating the pivot point limits the performance of the system, insofar as to place the end actuator according to the needs of the operation it is necessary to calculate its position according to the additional support point given by the pivot point or insertion point. In the present invention, instead of fixing the relative position of the robot and the patient using slips or physical fixings of the robot to the operating table, or doing a previous calculation of the location of the pivot point, there is a method for calculating the movement that is to be given to the camera for it to reach the desired location which avoids both previous alternatives. Thus, the integration of the system in the operating room is facilitated (as it does not require the modification of the operating table to fix it) and the safety of the operation is increased (since it can be easily and rapidly removed if necessary, and it eliminates the vulnerability of the system in case of changes of position of the patient in the operating table, or changes in the insertion point of the camera needed during the operation).

The whole system is electrically powered through batteries, and has wheels with brakes which enable to immobilize the assembly during the operation, and at the same time facilitate its movement using a handle, either to transport it or remove it from the operating table during surgery.

Brief description of the drawings

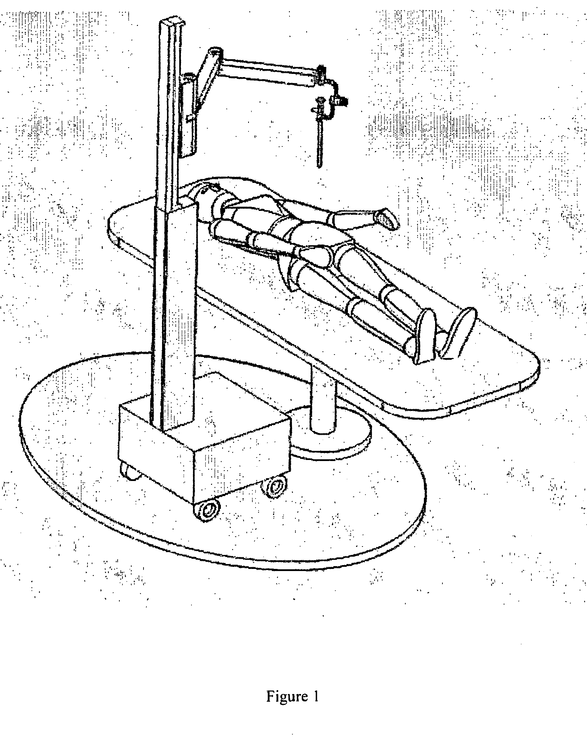

**Figure 1**: general schematic view of the robotic system for assisting in minimally-invasive surgery which can position a surgical instrument in response to orders from a surgeon, it is not attached to the operating table and does not require pre-calibration of the insertion point. A simplified representation of the manipulative surgical devices is shown, with a surgical instrument installed using an adaptor, placed next to the operating table.

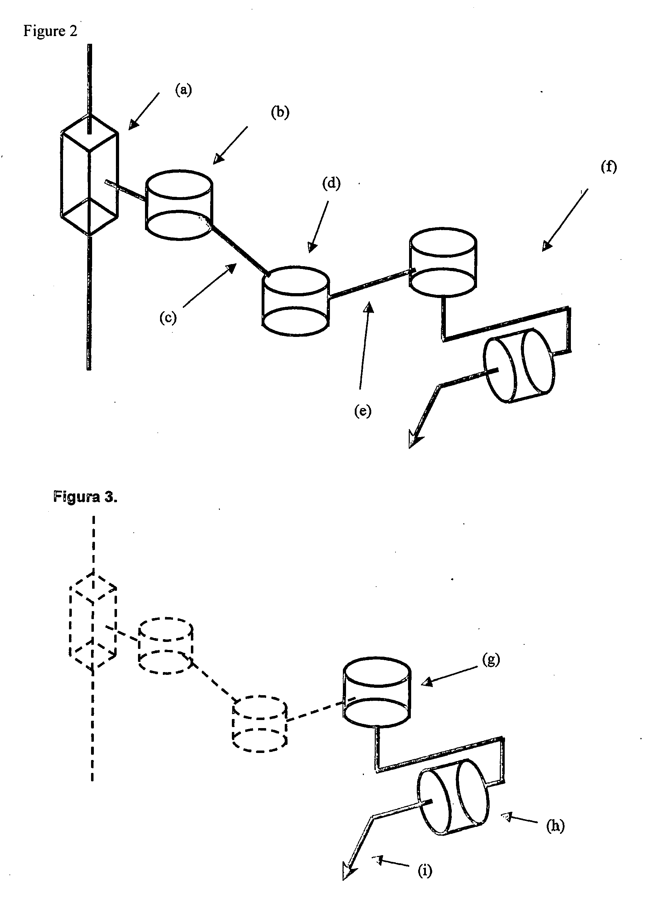

**Figure 2**: joint scheme of the robotic arm, including the end actuator that holds the surgical instrument (represented with an arrow).

**Figure 3:** joint scheme of the end actuator that holds the surgical instrument. It includes two passive joints (g) and (h) which enable the surgical instrument (i) to move with two degrees of freedom. The first is arranged with its rotation axis parallel to those of the operated joints of the manipulative device, and the second with its rotation axis perpendicular to them.

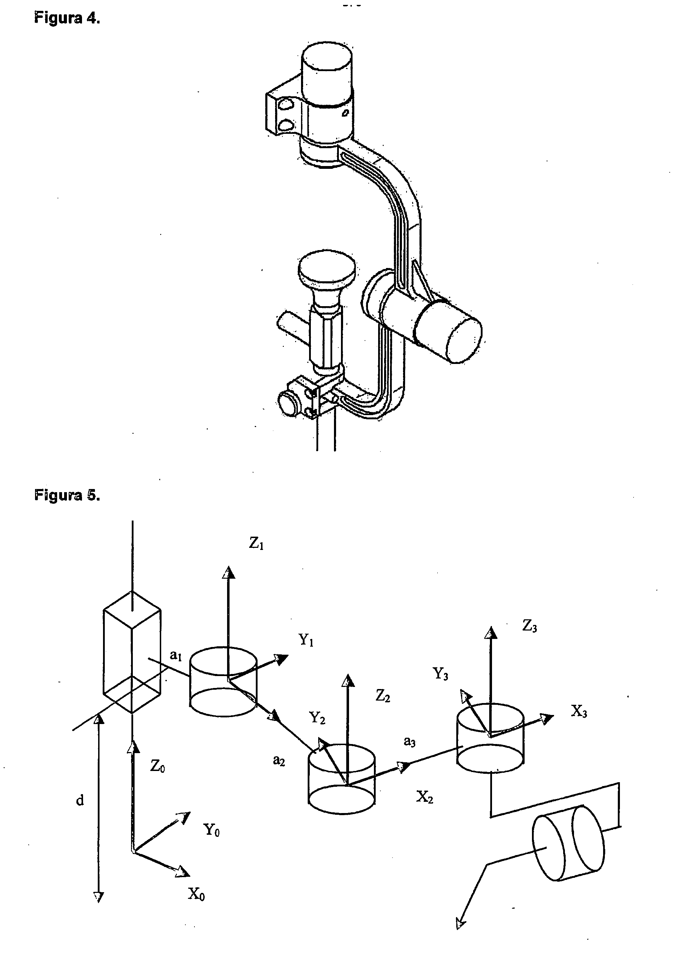

**Figure 4:** adaptor of the surgical instrument in the end actuator. In the scheme, it is represented holding the optic (j) (not represented in its entire length) of a laparoscopic camera. The passive joints are designated as (g) and (h).

**Figure 5:** joint scheme of the robotic arm including the representation of the reference systems used to obtain a direct kinematic model from it. These systems have been chosen and placed according to the Denavit-Hartenberg method.

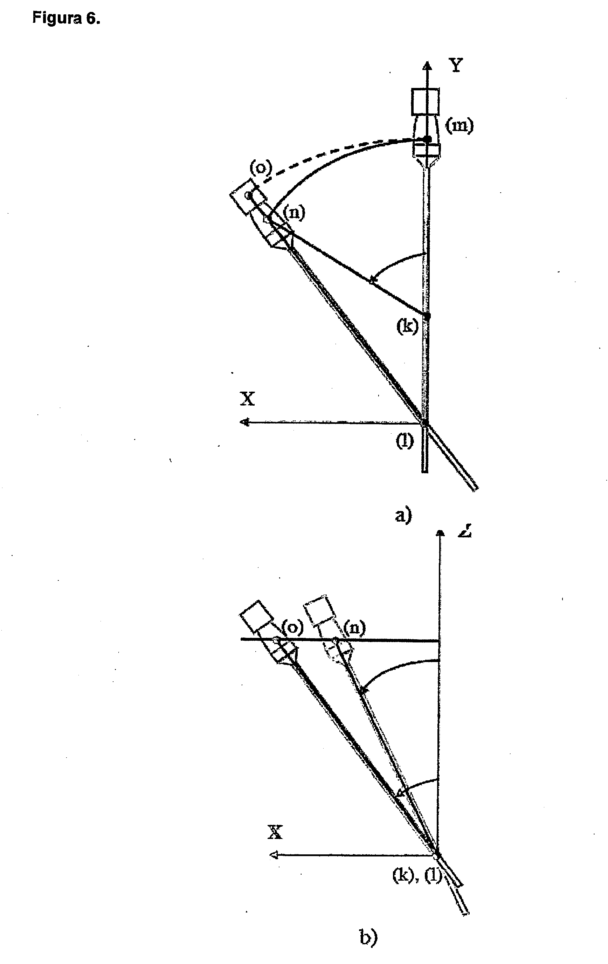

**Figure 6:** representation of the effect produced if the location of the insertion point of a surgical instrument is not precisely known when its movement is planned in a robot with passive joints in the end actuator. Figure 6a) shows a plan view, and figure 6b) shows a lateral view. If the real insertion point (I) is miscalculated, and (k) is used in its place, the system starts from the point (m) to reach (n) as a desired objective, but being the real support point and therefore the real radius of gyration different from the ones considered to calculate the final position, the objective position calculated is not (n) but (o). This results in inadequate orientation and final position which diminish the performance of the system.

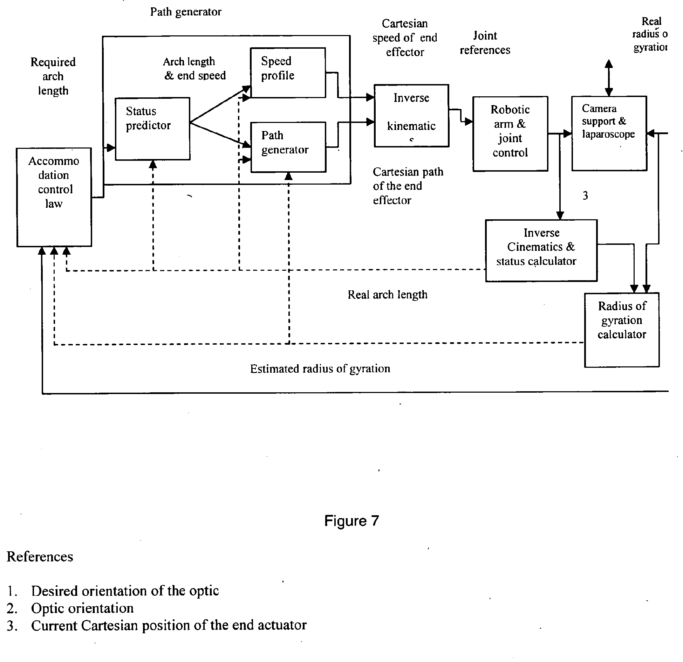

**Figure 7:** block diagram of the method used in the present invention to dynamically calculate the objective position of the instrument during the passage from the initial position to the final position refeeding the position of the insertion point of the instrument so that there is no need to anchor the robot to the operating table or pre-calibrate it. From the desired orientation an accommodation control law calculates the arch length required at every instant of the transit between the initial position and the final position to reach the objective, also using the real arch length covered, the real orientation and the calculated radius of gyration (that is to say, the estimated distance according to the axis of the instrument to the insertion point), all of it, at every instant. This required arch is used in a dynamic path generator together with the estimated radius of gyration, so that at every instant joint references are generated for the motorized joints of the robotic arm, which are corrected according to the new calculations, which finally gives movement to the attached instrument which through the passive joints reaches the desired orientation.

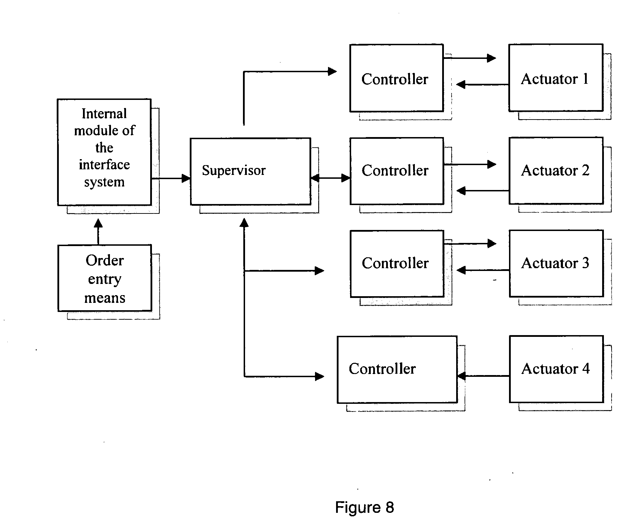

**Figure 8:** block diagram of the control architecture used in the controller of the robotic arm of the present invention. The surgeon gives orders through means arranged to that end, which send them to an internal module of the interface system annexed to the controller of the robotic arm. This element transmits these orders to the supervisor, who calculates, according to the method described, the final position that the motorized joints of the robot have to adopt for the instrument held to reach the desired location. This position is sent to the controllers which are in charge of making each joint reach its objective. Together with the controllers there are methods for receiving the signals sent by the sensors of the end actuator, and sending them to the supervisor.

Preferred embodiments of the invention

The system comprises a robotic arm with three active degrees of freedom (that is to say, motorized), an end actuator which incorporates two passive degrees of freedom (without engines) and especially designed to fasten a standard camera of the kind normally used in laparoscopic surgery, a controller for the robotic arm integrated in its structure and which can implement a method to calculate the movement needed to be given to the camera for it to reach the desired location without need to previously calibrate the device or fix it to the operating table, and an interface system which enables to interpret a surgeon's orders and transmit them to the aforementioned controller, as well as provide system information to the user. The interface system in turn comprises an internal module, annexed to the controller, and means for entering orders which allow the surgeon to move the surgical instrument, through the robot, as s/he desires. The internal module generates the movement orders appropriate for the robot in response to the surgeon's commands, which are expressed through the entry means.

The robotic arm is installed on a mobile mounting with wheels which can be placed next to the operating table (Figure 1). The mounting has brakes or an analogous system which enables to immobilize it, and it also has a handle to move it easily and direct the robotic arm over the mobile mounting. Both the mounting and the arm itself can be covered with a sterile cover. On the base of said mounting there is a controller and a battery system which provides electric power to the assembly. In the structure of the manipulative arm there is the internal module of the interface system, which is in charge of generating movement orders appropriate for the robot to move the attached surgical instrument according to the desires stated by the surgeon using the means for entering orders. The assembly of the controller, batteries and internal module of the interface system are located in a box attached to the base of the robotic arm which can be completely dismantled and replaced by another one with similar connections with the rest of the robot to facilitate repairs, or change between different embodiments of the aforementioned elements.

The robotic arm (Figure 2) has a first prismatic joint (a). This prismatic joint moves in the Z axis of a first system of coordinates. A second revolute joint (b) moves the second member of the robot (c) in a parallel plane to the X-Y plane defined by the first system of coordinates.

The second member of the robot (c) is joined to a third joint (d), also a revolute joint, which moves the third member of the robot (e) in the same plane of the joint (b). This member (c) is joined to an end actuator (f), which enables to securely fasten an endoscope, and which adds two passive degrees of freedom to the assembly. These two passive degrees of freedom enable to securely use the endoscope, as they prevent the patient's skin from being strained when this instrument is moved, even in spite of the fact that an error of movement may be made. In the preferred embodiment, the joint (a) is operated through an engine coupled to the axis of a vertical lineal displacement device, which has a mobile gib to which the rest of the robotic arm is fixed; the joint (b) can be operated directly through an engine coaxial with the joint axis; while the joint (d) moves through a transmission belt which transmits the movement from the engine located on the same axis that the one of the joint (b), to the joint (d). Said transmission belt is hidden inside the structure of the second member of the robotic arm, so that it is not accessible from the exterior.

The aforementioned end actuator is fixed to the wrist of the robot (Figure 3). It comprises two passive joints (g) and (h) which enable the surgical instrument to move with two degrees of freedom when it is not inserted on the patient's abdomen. These two degrees of freedom grant the system more security, insofar as they allow the pivot point to be established naturally by the movement of the instrument, in the same way as when it is operated by a person. Besides, both joints have means to find out the magnitude of the angle they have turned (not shown on the scheme of figure 3), so that this value can be available for the system. Those sensors can be, for example, potentiometers or angle encoders, but always of a resolution that allows to apply the method to calculate the movement to be imparted to the camera for it to reach the desired location, which is described later. There can be two sensors per axis, which allows for redundancy in the measurements, either to find the average of the two of them or to detect the possible failure of a sensor. Both passive joints are arranged so that the first (g) one is parallel to the two joints of the arm revolution, while the second (h) one is perpendicular to the first one, and both of them are cut in the axis of the surgical instrument attached to the robot. This disposition enables to dispense with a third revolute joint and its actuator, needed to give the system the capacity to reach the entire work space of the surgical instrument with the adequate orientation. This results in greater simplicity and economy of the system. Furthermore, the adaptor holding the surgical element has means to securely and firmly fasten said element, which at the same time allow to rapidly remove the optic from the endoscope without any tools.

Figure 4 shows a possible embodiment of the adaptor, where the passive joints. (g) and (h) and the optic (j) of a laparoscopic camera can be appreciated.

To improve the security, the robotic arm has a space inside it for the entire wiring needed to make the system work to pass through it and to hide the connectors, so that nothing is hooked with other material from the operating room or other objects, or even people working in the operating room, which can cause the malfunctioning of the system.

In order to know the initial position of the robotic arm when it is connected, there are means that enable to identify said initial position of the joints without these means being an obstacle to the normal performance of the system or being accessible from the exterior. These means consist of, in the first (a) and second (b) motorized joints (prismatic and first revolute joint), in a sensor located in a parallel axis of the joint (or, in the prismatic (a), parallel to the axis of the actuator available), which detects part of a circular part located in a perpendicular plane to said axis and integral to the second member (c) of the robotic arm (which joins the second joint (b) with the third (d) one). In the preferred embodiment of the invention, said part consists of a disc with a part of it having a bigger radius than the rest, and said sensor is a presence detector, so that in part of the turning space of the joint said sensor detects said part having a bigger radius, and the rest does not detect anything. In other alternative embodiments of the invention, the sensor can be (for example, but not exclusively) an image sensor, a magnetic sensor or a contact sensor. Therefore, the part mounted on the joint axis must be appropriate for the characteristics of the given sensor, for example, and respectively, having a disc with two sectors of different colors, two materials of different magnetic properties or two different levels which make contact or do not make contact with the aforementioned contact sensor.

To identify the initial position of the joint, the arrangement described is combined with a method:

1) when the robotic arm is turned on, it is checked if the sensor detects the presence of the distinctive sector of the part mounted on the joint axis (in the preferred embodiment of the invention, the part of the disk having a bigger radius).
2) If detected, the joint is moved until it is no longer detected.
3) If not detected, the joint is moved until it is detected in the direction opposite to the one in which it moves in the other case.

With this method, and knowing the width of the distinctive sector of the part, it is possible to find the initial position of the robotic arm.

In the case that the third motorized joint (d) (second revolute joint), there is similar arrangement, but the part that detects the sensor is integrally fixed to a point in the transmission belt which goes from the engine of the third joint (d) to the joint axis thereof, and the sensor is fixed in a known location on the inside of the member (c) of the robot that joins the second and third joints operated ((b) and (d), respectively). Thus, when the robotic arm is connected, the third motorized joint (d) moves according to the method described before, and likewise, it is possible to know the initial position of the joint knowing the situation of the sensor and the integral element to the transmission chain. In the preferred embodiment of the invention, the element detecting the sensor is an L-shaped part with a side attached to the transmission belt, and the sensor is a presence detector.

The controller of the robotic arm is located on the base of the mobile mounting thereof. This controller receive signals coming from the position sensors located in each one of the active and passive joints of the robot (therefore including the end actuator), which enables to know its position at all times through a direct kinematic model from it, obtained establishing several systems of coordinates along the robotic arm according to the Denavit-Hartenberg convention (see Figure 5).

Likewise, using the inverse kinematic model of the robotic arm and a path planning (in position, speed and acceleration), and the information provided by the angular sensors located in the robot, the controller mentioned before calculates the operations needed to reach the desired position of the attached instrument according to the surgeon's commands, stated using a method later described.

The controller of the robotic arm must have enough capacity to implement a method to dynamically calculate the objective position of the instrument during the passage from the initial position to the final position, which eliminates the need for previous calibration or fixing the assembly to the operating table. Furthermore, the sensors mounted on the passive joints of the end actuator must have enough resolution for the aforementioned method to be effectively applied.

Ideally, if the position of the insertion point is known with accuracy, once the surgeon gives an order, the desired position of the outer end of the instrument is calculated according to the current orientation and position of the instrument and the radius of gyration (that is to say, the distance along the tool axis from the outer end to the insertion point), and once the objective position is reached, through the passive joints and the support point determined by the insertion point of the tool, said tool adopts the desired orientation and position. However, if the insertion point is not precisely known, this results in the situation shown in Figure 6. Figure 6a shows a plan view and Figure 6b shows a lateral view. The real insertion point (I) is miscalculated and instead, (k) is used. The systems starts from point (m) to reach (n) as the desired objective, but being the support point and radius of direction different, the objective position calculated is not (n) but (o), giving the instrument a turning calculated using the incorrect radius of gyration. These results in an inadequate orientation and final position which diminish the performance of the system, or force to physically fix the robotic arm to the operating table or initially calibrate the location of the insertion point with respect to the robotic arm.

In the present invention, a method is used to dynamically calculate the objective position of the instrument during its transit from the initial position to the final position refeeding the position of the insertion point (I) so as to eliminate errors in reaching the objective position and orientation without anchoring the device to the operating table or calibrating it before the robotic arm takes part in the surgery.

At the initial moment, the instrument is inserted in an insertion point (I) (fulcrum) and its outer end is in a known position with an orientation that is also known. When the surgeon gives an order, this results in a new desired position of the internal end, which in turn implies a new desired position of the end outside the patient and a new desired orientation thereof. Reaching them depends on how precisely the position of the insertion point (I) is known. According to the diagram of Figure 7, from the desired orientation a control accommodation law calculates the required length of the arch at every instant of the passage from the initial position to the final one for reaching the objective, also using the real arch length covered, the real orientation and the estimated radius of gyration (that is to say, the estimated distance according to the axis of the instrument to the insertion point (I)), all of this at every instant. This required arch is used in a dynamic path generator with an estimated radius of gyration, so that at every instant joint references are created for the motorized joints of the robotic arm which are corrected according to the new calculations, which finally imparts a certain movement of the attached instrument which through passive joints reaches the orientation desired.

The aforementioned method is now described in greater detailed. The entry to the system is the desired orientation of the instrument, which can be either according to a vertical spin axis or according to the horizontal spin axis (in either case only one of the angles, since they are problems that can be laid off and they are calculated separately using the same method). To this desired orientation a temporal law of first order is associated according to the length in a straight line covered by the end of the instrument to reach such orientation:

$$\dot{L}(t) = -\frac{1}{\tau}L(t) + \frac{K}{\tau}u(t),$$

where L(t) is the straight line length covered by the end of the instrument according to the time, *T* is the time constant of the system, K is the static gain of the system and u(t) is the desired arch length, which is a function of the orientation of the instrument and its radius of gyration (that is to say, the distance from the point where the axes of the passive joints intersect in the instrument to the insertion point of said instrument in the patient).

The aforementioned expression, in discrete time, has the following form:

$$L(k+1) = e^{-T/\tau}L(k) + K(1 - e^{-T/\tau})u_r,$$

where L(k) is the straight line length covered by the end of the instrument in the instant K, T is the time constant of the system, T is the sampling period of the discretization, K is the static gain of the system and $u_r$ is the arch length to be covered to reach the desired orientation, which is a function of the orientation of the instrument and the radius of gyration thereof (that is to say, the distance from the point where the axes of the passive joints intersect in the instrument to the insertion point of said instrument in the patient). This late radius of gyration is not known, and this affects the precision of the orientation and position with which the attached instrument is located.

To eliminate the error made by the aforementioned lack of knowledge a control law is established in the space of states with two state variables: the length L covered in a straight line by the end of the instrument and a state variable which represents the angular error made. To obtain said control law, the following state equation is used:

$$\begin{pmatrix} L(k+1) \\ v(k+1) \end{pmatrix} = \begin{pmatrix} e^{-T/\tau} & 0 \\ -1/k_p & 1 \end{pmatrix} \cdot \begin{pmatrix} L(k) \\ v(k) \end{pmatrix} + \begin{pmatrix} K \cdot (1 - e^{-T/\tau}) \\ 0 \end{pmatrix} \cdot u(k),$$

where L(k) is the length covered in a straight line by the end of the instrument in the instant k, v(k) is the angular error made in the orientation of the instrument in the k instant, T is the time constant of the system, T is the period, of sampling of the discretization, $k_p$ is the radius of gyration, K is the static gain of the system, and u(k) is the arch length to be covered to reach the desired orientation at every instant k.

From this point, the following accommodation control law is obtained:

$$u(k) = k_p \cdot \left( \begin{pmatrix} r(k) \\ 0 \end{pmatrix} - H(k) \cdot \begin{pmatrix} L(k) \\ v(k) \end{pmatrix} \right),$$

where u(k) is the arch length to be covered to reach the desired orientation at every instant k, $k_p$ is the radius of gyration, with $k_p$ value = Þ (where Þ is the estimated radius of gyration) in the case that the orientation according to the horizontal axis and with $k_p$ value = sen(β) (where β is the angle of the instrument with respect to the vertical) in the case that the orientation according to the vertical axis, r(k) is the desired orientation at every instant k, modified at every instant according to a trapezoid profile for the first order system described according to the aforementioned temporal law not to evolve sharply, H(k) is a matrix of selected gains for the system to reach its objective, L(k) is the straight line length covered by the end of the instrument in the instant k, and v(k) is the angular error made in the orientation of the instrument in the instant k. Therefore, the accommodation control law needs the orientation of the optic (measured using sensors mounted on the end actuator) and the orientation desired to calculate the error, the estimated radius of gyration Þ and the arch length covered L(k).

The result of the accommodation control law is a required arch length, which is used together with the estimation of the real arch length covered until the moment to calculate the arch length and speed, both of which referring to the end of the instrument, through a predictor of the state constituted by the equation of state previously shown:

$$\begin{pmatrix} L(k+1) \\ v(k+1) \end{pmatrix} = \begin{pmatrix} e^{-T/\tau} & 0 \\ -1/k_p & 1 \end{pmatrix} \cdot \begin{pmatrix} L(k) \\ v(k) \end{pmatrix} + \begin{pmatrix} K \cdot (1 - e^{-T/\tau}) \\ 0 \end{pmatrix} \cdot u(k),$$

where the different magnitudes have the same meaning previously explained.

This double result is used as an entrance to calculate the speed profile and the path to be followed by the end actuator, in both cases also using as an entrance the estimation of the real arch length until the moment, and additionally, in the case of the path, with the support of the estimated radius of gyration Þ. Using the inverse kinematic model of the robotic arm, from the Cartesian speed and path of the end actuator obtained the joint references are derived, which are conducted to the joints of the robotic arm to reach the objective location. This movement moves the end actuator, which through the passive joints and the support point that the (real) insertion point is, makes the attached instrument reach a new orientation. At the same time as the new joint references are sent to the joints of the robotic arm, through said joint references and the direct kinematic model of the robotic arm, and according to the equation of the state previously shown (which here works as a state estimator), the estimation of the real arch covered so far is obtained in a first step, and in the next step, with the help of a radius of gyration estimator, said estimated radius of gyration Þ, in this case also using as an entrance the real orientation of the optic through the sensors of the end actuator.

The robotic arm controller has a hierarchical architecture (see Figure 8) comprising a controller for each active degree of freedom and a supervisor which calculates, according to the orders given by the surgeon and received through the interface system and according to the previously described method, the final position that the motorized joints of the robot must adopt for the attached instrument to reach the desired location, as well as the series of positions which need to be covered for the attached instrument to describe a straight line from its initial position and orientation to the final ones. This series of joint positions is sent to the controllers in charge of making each joint reach its objective. The communications between the different elements that intervene in the controller of the robotic arm are attained through a proprietary bus. In the preferred embodiment, the controller is implemented through an electronic circuit for the level of supervision and other three electronic circuits, alike and interchangeable, for the controllers of the motorized joints. Together with the controllers there are also means for receiving the signals sent by the different sensors with which the end actuator is equipped, and sending them to the supervisor. These means, in the preferred embodiment, are also implemented through an electronic circuit.

Annexed to this controller, and even also on the base of the robotic arm, there is a module which receives the surgeon's orders and generates, in response to them, the movement orders adequate for the robot. Likewise, this internal module is in charge of the communication with the means for entering orders. The embodiment of this internal module can vary according to the orders entry means chosen by the user. In the preferred embodiment it consists of an electronic circuit specialized in charge of recognizing the spoken orders given by the user through a microphone, but it can also take the shape of a program which is executed in a machine with a general purpose, such as a digital signal processor, a personal digital assistant (PDA), or a minicomputer, integrated in the mechanical structure of the arm.

Likewise, in another embodiment of the system in which oral orders are not chosen, or in which these are not the only possibility for the user to interact with the robot, the aforementioned module is in charge of controlling other order entry means, such as (but not exclusively) a touch screen. Both the signals of the aforementioned additional control means and the orders received through a voice interface are transformed in the internal module in instructions sent to the robot controller. These indicate the movements the arm needs to make.

As order entry means, in the preferred embodiment of the system there is a joystick or control lever attached to the robotic arm and a microphone, preferably wireless (although it can also be a conventional one), which is located in the structure of the robotic arm or held by the surgeon. But it can also have other entry means, such as a touch screen attached to the structure of the robot using an articulate mechanism which enables the user to locate it in a more convenient position. In this screen there is a laparoscopic image, and on top of it different kind of information can be shown, such as help marks for the operation, status of the system, information about the performance of other equipment of the operating room (which can also be controlled from the touch screen), or any other information of interest. Other control means can comprise, for example (but not exclusively), a master manipulator which reproduces the form of the surgical instrument held by the robot, so that the modification of the position of said master manipulator implies, through the appropriate conversion made by the internal module of the interface system, a similar movement in the surgical instrument attached to the manipulator. This master manipulator can have means which allow the user to specify a gain K or an attenuation $K^{-1}$ in the translation of the movements, so that a movement of a length L, in a determined direction, moves the real tool a magnitude $K \cdot L$ or $K^{-1} \cdot L$, respectively, in the corresponding direction. Another possibility, although limited to the preparation of the operation (with the surgical instrument not yet fixed to the end actuator) consists of moving the robotic arm directly with the hand until it is located in a more convenient point to fix the instrument to the robot, which is attained letting the second and third operated joints ((b) and (d), respectively) uncontrolled and regaining control with a specific order from the user when the instrument is fixed.

The internal module of the interface annexed to the controller is also in charge of presenting different information to the user, for example, but not exclusively, diagrams representing the real position of the surgical instrument or areas where movement is dangerous. Likewise, it is possible to keep record of the movements made by the robot, and the positions occupied by the system, enabling a later reconstruction of the words or sounds, if necessary. It can also return sound information, in the form of words or sounds, about the status of the system, which enables to set it up. Thus for example, it is possible to choose an initial configuration of "left arm" or "right arm" for the robotic arm according to what the operation to be performed advises, so that the third motorized joint (the second revolute joint) moves away from the surgeon and does not interfere with him/her. Another example consists of choosing, according to the surgeon's needs or preferences, the magnitude of the movement to be given to the end of the surgical instrument inside the patient in response to the orders given by the surgeon during the operation.

The assembly (the manipulative arm with the controller, the internal module of the interface system and the order entry means present in the operating room included) is battery operated, which gives it independence in the electric installation of the operating room where it is used, and simplifies its use and exploitation.

Industrial application

The system allows to apply the precision and safety features of robots to the field of surgery, specifically minimally-invasive surgery. When used to move a laparoscopic camera, a series of advantages are obtained such as a more stable image (without it affecting the operation time), better coordination among the medical staff and a reduction of the operation time (which in turn reduces the time the patient is under anesthesia). Furthermore, the use of a voice recognition system as a means for the surgeon to give orders to the robotic system enables him/her to handle the additional instrument without the help of an assistant, which enables to easily perform complex tasks within the techniques of minimally-invasive surgery. Thanks to the method used to calculate the following locations of the instrument, the robotic arm can be easily integrated and with a lower cost in the operating room as it is not necessary to modify the operating table (nor even change it) to anchor the robotic arm, and it is not necessary either to perform a calibration previous to the operation to find the location of the insertion point of the instrument, which shortens the duration of the operation and improves the system amortization since it enables the fast change of one surgical procedure to another even during the same operation. This quality of easiness and economy of integration is reinforced by being battery operated.

**Claims**

**1.** Robotic system for assisting in minimally-invasive surgery, which is not attached to the operating table and does not require pre-calibration of the insertion point, capable of positioning a surgical instrument in response to orders from a surgeon, **characterized in that** it uses a robotic arm with three active degrees of freedom and two passive ones (the three of them active and arranged in such a way that the first one is prismatic and the second and third ones are rotary, and they are found arranged with respect to one another as shown in Figure 2; and the two passive ones are rotary and perpendicular to one another, arranged as shown in Figure 3, an end actuator enables to hold the endoscopy required for the surgical procedure for it to be used in the system, a controller for the robotic arm integrated in the mechanical structure thereof and capable of implementing a method which calculates the movement to be given to the surgical instrument held for it to reach the desired location with precision without requiring to fix the system physically to the operating table and without needing previous calibration to estimate the location of the insertion point of the instrument, and an interface system to order the desired actions to the system, comprising also a module annexed to the robot controller, and means to give said orders, wherein the whole assembly is battery operated and set on a mobile mounting which has wheels with brakes or a similar device to enable to immobilize it safely, and the aforementioned method to calculate the movement to be given to the surgical instrument consists in:

    a) At the initial instant, the instrument is inserted into an entrance point (or fulcrum point) and its outer end is in a known position with a known orientation.
    b) When the surgeon gives an order, this results into a new desired position of the internal end, which in turn implies a new desired position of the end outside the patient and a new desired orientation thereof. Reaching them depends on how precisely the location of the insertion point is known.
    c) From the desired orientation an accommodation control law calculates the required arch length at every instant of the passage between the initial and final positions for the objective to be reached, also using the real arch length covered, the real orientation and the calculated radius of gyration (that is to say, the estimated distance according to the axis of the instrument to the insertion point), all of it, at every instant.
    d) This required arch is used in a dynamic path generator together with the estimated radius of gyration, so that at every instant joint references are generated for the motorized joints of the robotic arm, which are corrected according to the new calculations, which finally gives movement to the attached instrument which through the passive joints reaches the desired orientation.

**2.** Robotic system for assisting in minimally-invasive surgery, which is not attached to the operating table and does not require pre-calibration of the insertion point according to claim 1, wherein the assembly of the controller for the robotic arm, batteries and internal module of the interface system are located in a box attached to the base of the robotic arm which can be completely dismantled and replaced by another one with similar connections with the rest of the robot to facilitate repairs, or change between different embodiments of the aforementioned elements.

**3.** Robotic system for assisting in minimally-invasive surgery, which is not attached to the operating table and does not require pre-calibration of the insertion point according to claims 1 and 2, wherein the robotic arm has a space inside it for the entire wiring needed to make the system work to pass through it and to hide the connectors, so that nothing is hooked with other material from the operating room or other objects, or even people working in the operating room, which can cause the malfunctioning of the system.

4.  Robotic system for assisting in minimally-invasive surgery, which is not attached to the operating table and does not require pre-calibration of the insertion point according to claims 1, 2 and 3, **characterized in that** it comprises means that enable to identify said initial position of the robotic arm when it is connected, without these means being an obstacle to the normal performance of the system or being accessible from the exterior, and wherein these means consist of, in second (b) motorized joint (first revolute joint), in a sensor located in a parallel axis of the joint turn, which detects a part of a circular part located in a perpendicular plane to said axis and integral to the second member (c) of the robotic arm (which joins the second joint (b) with the third (d) one), and in the case of the third motorized joint (d) (second revolute joint), there is a similar arrangement but the part which detects the sensor is integrally fixed to a point in the transmission belt which goes from the engine of the third joint (d) to the joint axis thereof, and the sensor is fixed in a known location of the inside of the robot member which joins the second and third operated joints, and so knowing the location of the element that detects the sensor and the location of the sensor itself; the location of the joint can be found using the following method:

    • when the robotic arm is turned on, it is checked if the sensor detects the presence of the distinctive sector of the part mounted on the joint axis (in the preferred embodiment of the invention, the part of the disk having a bigger radius).
    • If detected, the joint is moved until it is no longer detected.
    • If not detected, the joint is moved until it is detected in the direction opposite to the one in which it moves in the other case.

5.  Robotic system for assisting in minimally-invasive surgery, which is not attached to the operating table and does not require pre-calibration of the insertion point according to claim 4, wherein the means to know the initial position of the robotic arm consist, in the second motorized joint (b), of a presence detector and a disk with a part having a bigger radius than the rest, and a third (d) motorized joint, the element detecting the sensor is an L-shaped part with a side attached to the transmission belt, and the sensor is a presence detector.

6.  Robotic system for assisting in minimally-invasive surgery, which is not attached to the operating table and does not require pre-calibration of the insertion point according to claims 1, 2, 3 and 5, wherein the robotic arm controller has a hierarchical architecture comprising a controller for each active degree of freedom and a supervisor which calculates, according to the orders given by the surgeon and received through the interface system, the final position that the motorized joints of the robot must adopt for the attached instrument to reach the desired location, as well as the series of positions which need to be covered for the attached instrument to describe a straight line from its initial position and orientation to the final ones and this series of joint positions is sent to the controllers in charge of making each joint reach its objective, and wherein the communications between the different elements that intervene in the controller of the robotic arm are attained through a proprietary bus.

7.  Robotic system for assisting in minimally-invasive surgery, which is not attached to the operating table and does not require pre-calibration of the insertion point according to claim 6, wherein the controller is implemented through an electronic circuit for the level of supervision and other three electronic circuits, alike and interchangeable, for the controllers of the motorized joints, and together with them there is also a fourth electronic circuit in charge of receiving the signals sent by the different sensors with which the end actuator is equipped, and sending them to the supervisor.

8.  Robotic system for assisting in minimally-invasive surgery, which is not attached to the operating table and does not require pre-calibration of the insertion point according to claims 1, 2, 3, 4 and 6, wherein a joystick or control lever attached to the robotic arm and a microphone are used as means to enter orders.

9.  Robotic system for assisting in minimally-invasive surgery, which is not attached to the operating table and does not require pre-calibration of the insertion point according to claim 8, wherein the module annexed to the controller in charge of receiving orders from the surgeon through the order entry means can return sound information, in the form of words or sounds, about the status of the system, which enables to set it up.

10. Robotic system for assisting in minimally-invasive surgery, which is not attached to the operating table and does not require pre-calibration of the insertion point according to claims 1, 2, 3, 4 and 6, wherein as entry means there is a touch screen attached to the structure of the robot using an articulate mechanism which enables the user to locate it in a more convenient position and where there is a laparoscopic image, and on top of it different kind of information can be shown, such as help marks for the operation, status of the system or information about the performance of other equipment of the operating room.

**11.** Robotic system for assisting in minimally-invasive surgery, which is not attached to the operating table and does not require pre-calibration of the insertion point according to claims 1, 2, 3, 4 and 6 wherein as entry means there is a master manipulator which reproduces the form of the surgical instrument held by the robot, so that the modification of the position of said master manipulator implies, through the appropriate conversion made by the internal module of the interface system, a similar movement in the surgical instrument attached to the manipulator.

**12.** Robotic system for assisting in minimally-invasive surgery, which is not attached to the operating table and does not require pre-calibration of the insertion point according to claims 1, 2, 3, 4 and 6, wherein during the preparation for the operation (and before fixing the surgical instrument to the end actuator) there is the direct handling of the manipulative arm as an entry means to take it to the most convenient point to fix the instrument to the end actuator.

Figure 1

Figure 2

(a)

(b)

(d)

(f)

(c)

(e)

Figura 3.

(g)

(h)

(i)

**Figura 4.**

**Figura 5.**

**Figura 6.**

a)

b)

Figure 7

References

1. Desired orientation of the optic
2. Optic orientation
3. Current Cartesian position of the end actuator

Figure 8

# INTERNATIONAL SEARCH REPORT

| | |
|---|---|
| International application No. | |
| PCT/ ES 2007/000442 | |

**A. CLASSIFICATION OF SUBJECT MATTER**

see extra sheet

According to International Patent Classification (IPC) or to both national classification and IPC

**B. FIELDS SEARCHED**

Minimum documentation searched (classification system followed by classification symbols)

A61B17,19, A61B1

Documentation searched other than minimum documentation to the extent that such documents are included in the fields searched

Electronic data base consulted during the international search (name of data base and, where practicable, search terms used)

CIBEPAT,EPODOC,WPI,LATIPAT

**C. DOCUMENTS CONSIDERED TO BE RELEVANT**

| Category* | Citation of document, with indication, where appropriate, of the relevant passages | Relevant to claim No. |
|---|---|---|
| A | WO 2006016390 A1 (CALABRIAN HIGH TECH SRL) 16.12.2006, the whole document. | 1 - 12 |
| A | WO 2006052375 A1 (LIPOW) 18.05.2006, the whole document. | 1 - 12 |
| A | WO 2006079108 A1 (INTUITIVE SURGICAL) 27.07.2006, the whole document. | 1 - 12 |
| A | US 2005027397 A (NIEMEYER) 03.02.2005, the whole document. | 1 - 12 |
| A | US 2005256371 A (SCHARA et al.) 17.11.2005, the whole document. | 1 - 12 |
| A | WO 2004032752 A1 (CENTRO DE INVESTIGACIóN Y DE ESTUDIOS AVANZADOS DEL INSTITUTO POLITéCNICO NACIONAL) 22.04.2004, the whole document. | 1 - 12 |

☒ Further documents are listed in the continuation of Box C.     ☒ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance. | |
| "E" earlier document but published on or after the international filing date | |
| "L" document which may throw doubts on priority claim(s) or which is cited to establish the publication date of another citation or other special reason (as specified) | "X" document of particular relevance; the claimed invention cannot be considered novel or cannot be considered to involve an inventive step when the document is taken alone |
| "O" document referring to an oral disclosure use, exhibition, or other means | "Y" document of particular relevance; the claimed invention cannot be considered to involve an inventive step when the document is combined with one or more other documents , such combination being obvious to a person skilled in the art |
| "P" document published prior to the international filing date but later than the priority date claimed | |
| | "&" document member of the same patent family |

| Date of the actual completion of the international search | Date of mailing of the international search report |
|---|---|
| 26 November 2007     (26.11.2007) | (28/11/2007) |
| Name and mailing address of the ISA/ O.E.P.M. Paseo de la Castellana, 75 28071 Madrid, España. Facsimile No. 34 91 3495304 | Authorized officer A. Cardenas Villar Telephone No. +34 91 349 53 93 |

Form PCT/ISA/210 (second sheet) (April 2007)

EP 2 047 805 A1

## INTERNATIONAL SEARCH REPORT

| | International application No. |
|---|---|
| | PCT/ES 2007/000442 |

| C (continuation). | DOCUMENTS CONSIDERED TO BE   RELEVANT | |
|---|---|---|
| Category* | Citation of documents, with indication, where appropriate, of the relevant passages | Relevant to claim No. |
| A | US 2004261179 A (BLUMENKRANZ) 30.12.2004, the whole document | 1 - 12 |
| A | ES 2200679 A (UNIVERSIDAD DE MáLAGA) 01.03.2004, the whole document. | 1 - 12 |
| A | ES 2181526 A(UNIVERSIDAD DE MáLAGA) 16.02.2003, the whole document. | 1 - 12 |

Form PCT/ISA/210 (continuation of second sheet) (April 2007)

20

| INTERNATIONAL SEARCH REPORT | International application No. |
| :---: | :--- |
| Information on patent family members | PCT/ ES 2007/000442 |

| Patent document cited in the search report | Publication date | Patent family member(s) | Publication date |
| :---: | :---: | :---: | :---: |
| WO 2006016390 A | 16.02.2006 | WO 2006016391 A<br>EP 1843713 A<br>EP 20050778903<br>EP 1843876 A<br>EP 20050778744 | 16.02.2006<br>17.10.2007<br>08.08.2005<br>17.10.2007<br>08.08.2005 |
| WO 2006052375 A | 18.05.2006 | US 2006155263 A | 13.07.2006<br>13.07.2006<br>13.07.2006 |
| WO 2006079108 A | 27.07.2006 | US 2006167440 A<br>US 2007156122 A<br>EP 1841379 A<br>EP 20060719471 | 27.07.2006<br>05.07.2007<br>10.10.2007<br>24.01.2006 |
| US 2005027397 A | 03.02.2005 | US 6493608 B<br>US 2003029463 A<br>US 6772053 B | 10.12.2002<br>13.02.2003<br>03.08.2004<br>03.08.2004<br>03.08.2004 |
| US 2005256371 A | 17.11.2005 | NONE | ------------ |
| WO 2004032752 A | 22.04.2004 | MX P<br>AU 2003267853 A | 28.04.2004<br>04.05.2004 |
| US 2004261179 A | 30.12.2004 | US 6246200 B<br>US 2001013764 A<br>US 6441577 B<br>US 6788018 B<br>US 6933695 B | 12.06.2001<br>16.08.2001<br>27.08.2002<br>07.09.2004<br>23.08.2005<br>23.08.2005 |
| ES 2200679 A B | 01.03.2004 | NONE | ------------ |
| ES 2181526 A B | 16.02.2003 | NONE | ------------ |

Form PCT/ISA/210 (patent family annex) (April 2007)

INTERNATIONAL SEARCH REPORT

International application No.

PCT/ ES 2007/000442

CLASSIFICATION OF SUBJECT MATTER

*A61B 17/00* (2006.01)
*A61B 19/00* (2006.01)

**EP 2 047 805 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5815640 A
- US 6371952 B
- ES 2150880
- EP 0571827 A
- ES 2181526
- WO 9403113 A
- WO 9729690 A
- US 5784542 A
- WO 9825666 A
- ES 2203318
- ES 2200679